# EUROPÄISCHE PATENTANMELDUNG

(11) **EP 0 881 488 A2**
(43) Veröffentlichungstag der Anmeldung: **02.12.1998**
(21) Anmeldenummer: 98890165.8
(22) Anmeldetag: 28.05.1998
(51) Int. Cl.: G01N 31/22, G01N 33/84, C07D 498/08

(54) **Verfahren zur Bestimmung eines Alkaliions**

(30) Priorität: 30.05.1997 AT 930/97
(71) Anmelder: AVL Medical Instruments, 8207 Schaffhausen (CH)
(72) Erfinder: Leiner, Marco Jean Pierre, 8045 Graz (AT); He, Huarui, Alpharetta, GA 30202 (US); Boila-Göckel, Andrei, 8010 Graz (AT)
(74) Vertreter: Schwarz, Albin, Dr. Kopecky & Schwarz Patentanwälte

(57) **Zusammenfassung**

Die Erfindung betrifft ein Verfahren zur Bestimmung eines Alkaliions in einer Probe, wobei das Alkaliion mit einer Verbindung, die einen luminophoren Rest und einen ionophoren Rest aufweist, in Kontakt gebracht wird, welcher ionophore Rest mit dem in der Probe enthaltenen Alkaliion reagiert, wobei der luminophore Rest seine Lumineszenzeigenschaften ändert, wonach die Lumineszenz gemessen und mit dem Meßergebnis das Alkaliion bestimmt wird, das dadurch gekennzeichnet ist, daß als Verbindung ein Diaza-Kryptand der allgemeinen Formel I 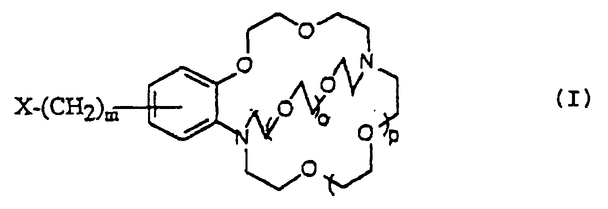 worin X der luminophore Rest ist, m die Zahl 0, 1 oder 2 ist, und o und p unabhängig voneinander die Zahlen 0, 1 oder 2 bedeuten, eingesetzt wird.

## Beschreibung

Die vorliegende Erfindung betrifft ein Verfahren zur Bestimmung eines Alkaliions in einer Probe, wobei das Alkaliion mit einer Verbindung (=Luminophor-Ionophor), die einen luminophoren Rest und einen ionophoren Rest aufweist, in Kontakt gebracht wird, welcher ionophore Rest mit dem in der Probe enthaltenen Alkaliion reagiert, wobei der luminophore Rest seine Lumineszenzeigenschaften ändert, wonach die Lumineszenz gemessen und mit dem Meßergebnis auf die Konzentration oder die Aktivität des Alkaliions geschlossen wird, d.h. das Alkaliion bestimmt wird. Die Erfindung betrifft auch Diaza-Kryptanden, die als Luminophor-Ionophore zur Bestimmung eines Alkaliions eingesetzt werden können.

Ein derartiges Bestimmungsverfahren beruht auf dem sogenannten "PET-Effekt". Darunter wird ein mittels Photonen induzierter Elektronen-Transfer (photoinduced electron transfer = PET) vom ionophoren Rest bzw. Ionophor auf den luminophoren Rest bzw. Luminophor verstanden, der zu einer Verringerung der (relativen) Lumineszenzintensität und der Lumineszenzabklingzeit des Luminophors führt. Die Absorptions- und die Emissionswellenlängen werden jedoch dabei im wesentlichen nicht beeinflußt (J.R. Lakowicz in "Topics in Fluorescence Spectroscopy", Band 4: Probe Design and Chemical Sensing; Plenum Press, New York & London (1994)).

Durch die Bindung von Ionen an den Ionophor wird der PET-Effekt teilweise oder vollständig blockiert, sodaß es zu einem Anstieg der Lumineszenz des luminophoren Restes kommt. Somit kann durch Messung der Änderung der Lumineszenzeigenschaften, d.h. der Lumineszenzintensität und/oder der Lumineszenzabklingzeit, auf die Konzentration oder die Aktivität des zu bestimmenden Ions geschlossen werden.

Aus der US-A - 5,516,911 sind Fluoreszenzindikatoren für die intracelluläre Kalziumbestimmung bekannt, welche fluoreszierende Substituenten tragen, die als optische Indikatoren wirken können.

Ein Verfahren der eingangs beschriebenen Art ist aus der US-A - 5,439,828 bekannt, wobei als Luminophor-Ionophor Diaza-Kryptanden verwendet werden, die mit fluoreszierenden Cumarinen als Fluorophor funktionalisiert sind und je nach Struktur für Lithium-, Natrium- bzw. Kaliumionen spezifisch sind. Es wird angegeben, daß diese Luminophor-Ionophore in pH-neutralen Probemedien verwendet werden können und in solchen Systemen auch bevorzugt eingesetzt werden.

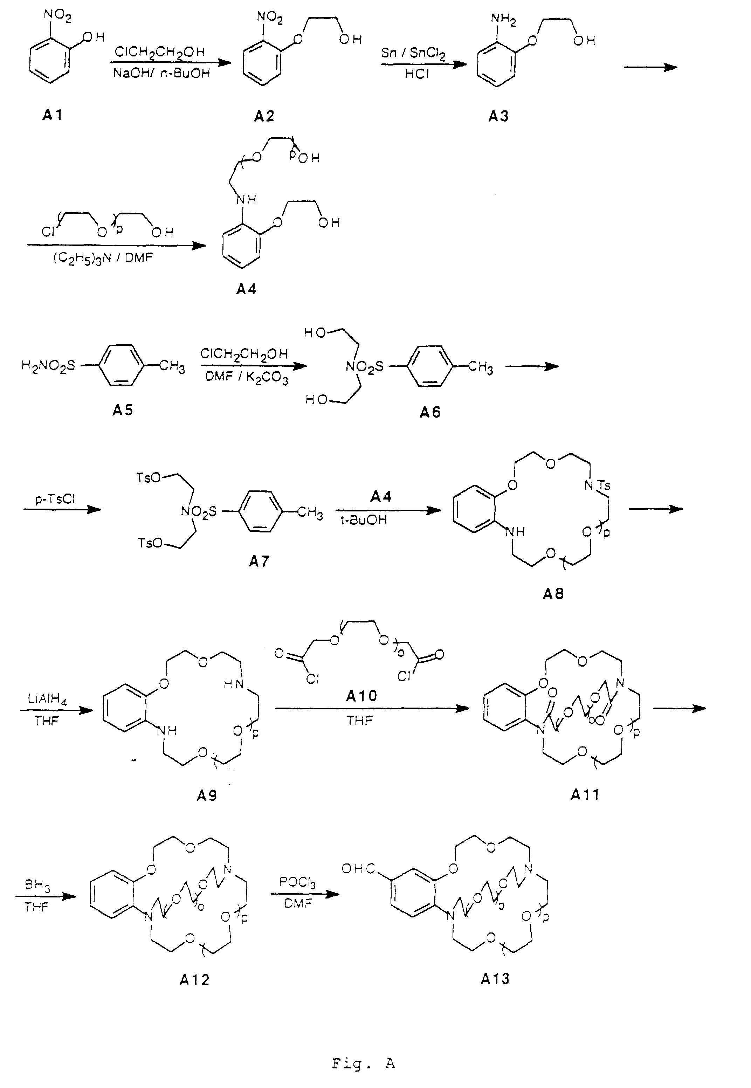

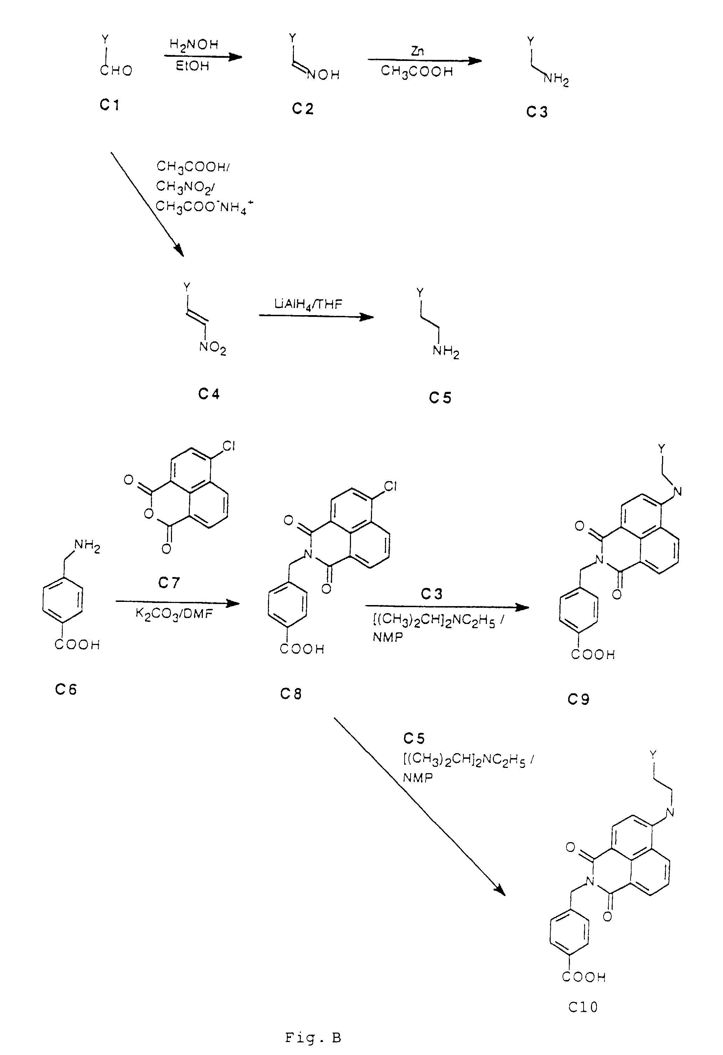

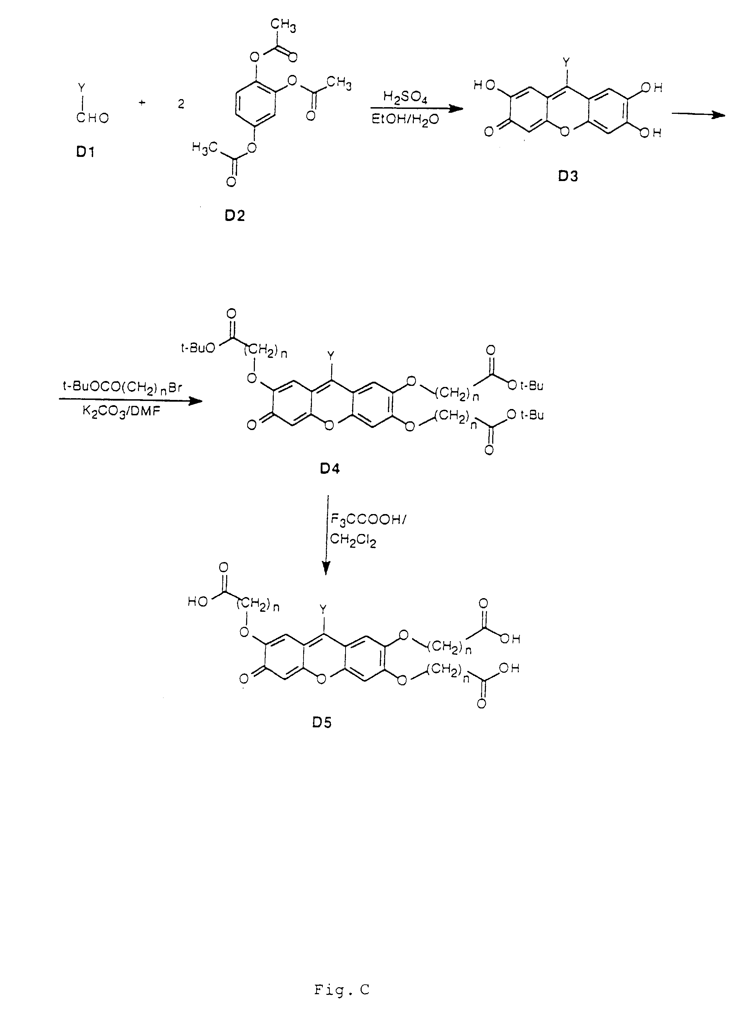

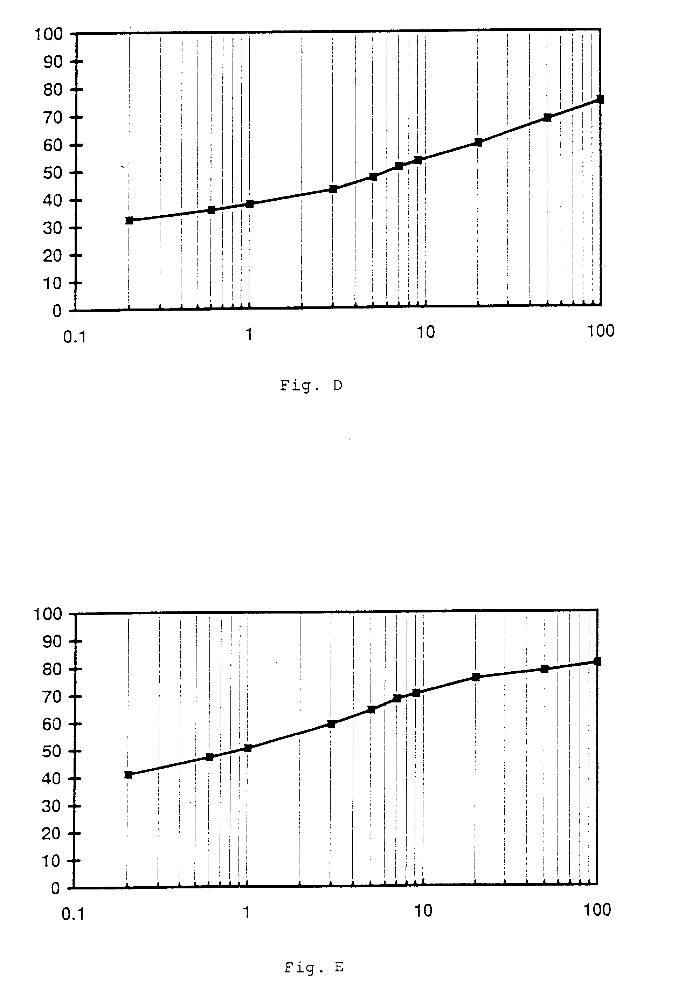

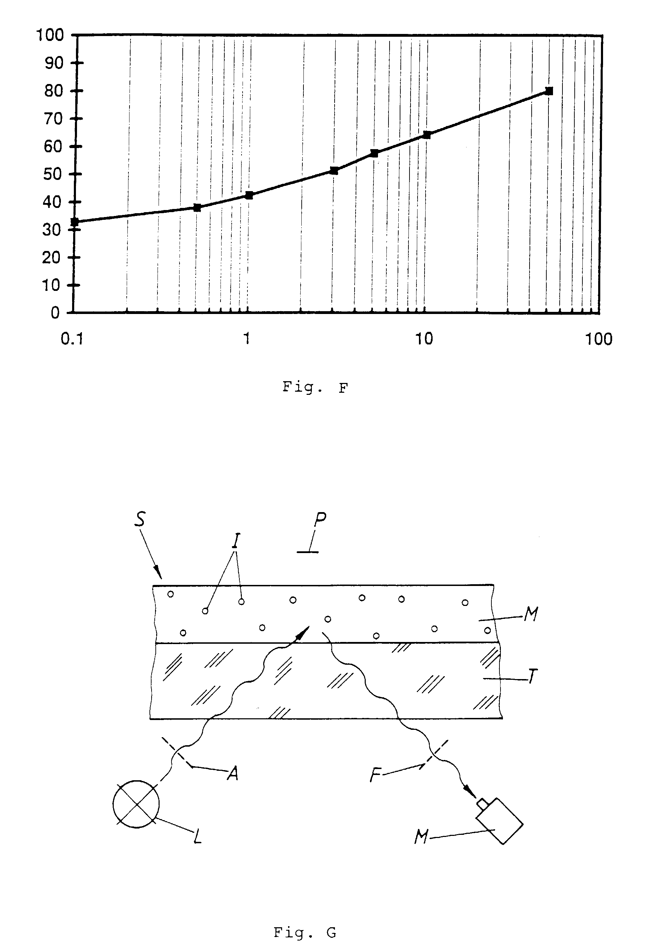

Untersuchungen (Frank Kastenholz, Inaugural-Dissertation, Universität zu Köln, 1993, Abb. 32, Seite 54) haben jedoch gezeigt, daß das Fluoreszenzsignal im physiologischen pH-Bereich signifikant vom pH-Wert der Probe abhängt und mit fallendem pH-Wert bereits ab einem pH von 7,4 stark ansteigt. Dies beeinträchtigt die Genauigkeit einer Bestimmung, die in biologischen Proben durchgeführt wird. Ferner haben die verwendeten Verbindungen den weiteren Nachteil, daß die verwendeten Cumarine Absorptionswellenlängen von etwa 336 nm aufweisen und daher nicht von kommerziellen LEDs angeregt werden können.

Diese Nachteile gelten auch für die in der US-A - 5,162,525 genannten Luminophor-Ionophore.

Aus Tetrahedron Letters, Band 31, Nr. 36, Seiten 5193-5196 (1990) sind Diaza-Kryptanden bekannt, bei denen beide Stickstoffatome an jeweils einen aromatischen Ring gebunden sind, d.h. Aryl-Stickstoffe bzw. Stickstoffe vom Anilintypus sind. Untersuchungen der Anmelderin haben gezeigt, daß sich diese Diaza-Kryptanden nicht zur Bestimmung von Kaliumionen eignen, wenn sie im physiologischen Konzentrationsbereich und bei physiologischen pH-Werten des Blutes (7,0-7,6) vorliegen.

Die vorliegende Erfindung stellt sich daher die Aufgabe, das vorbekannte Verfahren so zu verbessern bzw. Luminophor-Ionophore zur Verfügung zu stellen, die bei physiologischen pH-Werten keine signifikante Abhängigkeit der Lumineszenzeigenschaften vom pH-Wert der Probe zeigen und daher zur Bestimmung bei biologischen Proben geeignet sind.

Das erfindungsgemäße Verfahren soll ferner insbesondere bei Vorliegen physiologischer Alkaliionenkonzentrationen gut durchführbar sein, d.h. eine große Abhängigkeit des Lumineszenzsignals von der Konzentration des zu bestimmenden Alkaliions aufweisen.

Diese Aufgabe wird bei dem eingangs beschriebenen Verfahren dadurch gelöst, daß
als Verbindung (=Luminophor-Ionophor) ein Diaza-Kryptand der allgemeinen Formel I 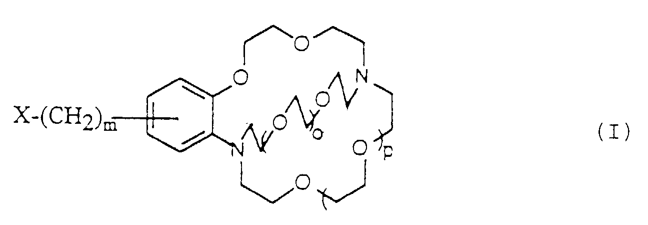 worin X der luminophore Rest ist, m die Zahl 0, 1 oder 2 ist, und o und p unabhängig voneinander die Zahlen 0, 1 oder 2 bedeuten, eingesetzt wird.

Die Diaza-Kryptanden mit der obigen allgemeinen Formel I sind neu. Es hat sich gezeigt, daß sich diese neuen Luminophor-Ionophore sehr gut zur Bestimmung von Alkaliionen bei physiologischen pH-Werten und bei physiologischen Konzentrationen eignen.

Es hat sich gezeigt, daß sich die erfindungsgemäßen Diaza-Kryptanden der allgemeinen Formel I insbesondere gut eignen, Lithiumionen im Konzentrationsbereich zwischen 0,30 und 2,1 mmol/l und Kaliumionen im Konzentrationsbereich zwischen 1,5 und 8,0 mmol/1 zu bestimmen.

Ohne an eine bestimmte Theorie gebunden zu sein, wird angenommen, daß bei den erfindungsgemäßen Diaza-Kryptanden die vorteilhaften Eigenschaften darauf zurückzuführen sind, daß im ionophoren Rest der eine Stickstoff ein Aryl-Stickstoff und der andere Stickstoff ein aliphatischer Stickstoff ist.

Als luminophorer Rest X eignen sich alle Reste, mit welchen sich in Kombination mit dem ionophoren Rest ein PET-Effekt erzielen läßt. Aus der Literatur ist eine Vielzahl von Resten bekannt, die in Kombination mit dem Ionophor den PET-Effekt ergeben oder sich prinzipiell dafür eignen. Durch Ankoppelung dieser vorbekannten Reste an den Benzolring der allgemeinen Formel I werden neue Verbindungen erhalten, an denen der Fachmann prüfen kann, ob sich ein PET-Effekt erzielen läßt. Die Ankoppelung kann zum Stickstoff in ortho-Position, in seinen beiden meta-Positionen und in para-Position erfolgen. Bevorzugt ist die para-Position.

Wie dem Fachmann bekannt ist, ist für das Zustandekommen eines PET-Effektes insbesondere eine elektronische Entkoppelung des Elektronendonors des ionophoren Restes vom elektronischen System des luminophoren Restes wesentlich. Diese elektronische Entkoppelung von ionophorem und luminophorem Rest kann bekanntermaßen dadurch erreicht werden, daß beide Reste entweder durch eine Spacer-Gruppe, also die (CH₂)ₘ-Kette mit m>0 oder - wenn m=0 - durch einen virtuellen Spacer (z.B. durch Verdrehung der Ebene des luminophoren Restes zur Ebene des Benzolringes) getrennt vorliegen. Die Funktion des Spacers besteht somit darin, einer Konjugation des Elektronensystems des ionophoren Restes mit dem Elektronensystem des luminophoren Restes entgegenzuwirken.

Die elektronische Entkoppelung ist z.B. daran erkenntlich, daß sich die Absorptions- und Emissionsspektren hinsichtlich ihrer Wellenlänge nicht wesentlich ändern.

Zur Bestimmung von Lithiumionen wird bevorzugt ein Diaza-Kryptand der allgemeinen Formel I eingesetzt, in welcher o und p die Zahlen 0 bzw. 0 bedeuten.

Zur Bestimmung von Natriumionen wird bevorzugt ein Diaza-Kryptand der allgemeinen Formel I eingesetzt, in welcher o und p die Zahlen 0 bzw. 1 bedeuten, oder die Zahlen 1 bzw. 0 bedeuten.

Zur Bestimmung von Kaliumionen wird bevorzugt ein Diaza-Kryptand der allgemeinen Formel I eingesetzt, in welcher o und p die Zahlen 1 bzw. 1 bedeuten.

Der luminophore Rest X in der allgemeinen Formel I ist bevorzugt
- ein Amino-Naphthalimid-Rest der allgemeinen Formel II 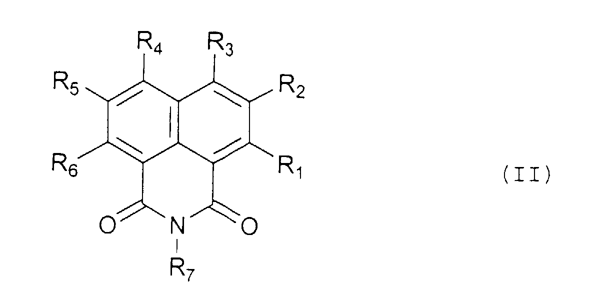 in welcher eine von R₁, R₂, R₃, R₄, R₅ und R₆ eine Gruppe -NH- ist, über welche X an die Gruppe -(CH₂)ₘ- der oben genannten Verbindung mit der allgemeinen Formel I gebunden ist, und die anderen und R₇ jeweils voneinander unabhängig Wasserstoff, eine lipophile oder hydrophile Gruppe oder eine reaktive Gruppe zur Kopplung an ein Polymer sind, oder
- ein Xanthenon-Rest der allgemeinen Formel III 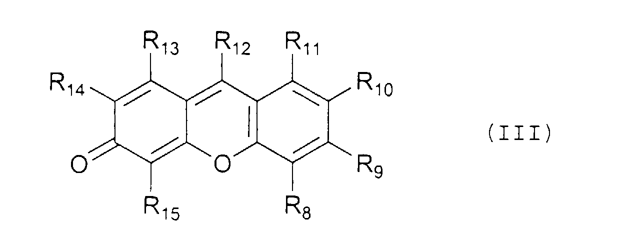 ist, in welcher eine von R₈, R₉, R₁₀, R₁₁, R₁₂, R₁₃, R₁₄ und R₁₅ für eine chemische Bindung steht, über welche X direkt (m=0) an den ionophoren Rest der oben genannten Verbindung mit der allgemeinen Formel I gebunden ist, und die übrigen für -OH, -OR₁₆, worin R₁₆ eine hydrophile oder eine lipophile Gruppe ist, -O-R₁₇-G, worin R₁₇ eine hydrophile oder eine lipophile Gruppe und G eine reaktive Gruppe zur Kopplung an ein Polymer ist, oder -(CH₂)ₙ-COOH, worin n eine ganze Zahl zwischen 0 und 17 ist, stehen.

Bevorzugt ist in der allgemeinen Formel II R₃ oder R₄ die Gruppe -NH-, über welche der luminophore Rest an die Gruppe -(CH₂)ₘ- der oben genannten allgemeinen Formel I gebunden ist.

Bevorzugt ist weiters in der allgemeinen Formel III R₁₂ eine chemische Bindung, über welche der luminophore Rest direkt (m=0) an den ionophoren Rest der oben genannten allgemeinen Formel I gebunden ist.

Als lipophile Gruppe eignen sich z.B. substituierte und unsubstituierte Alkylgruppen und Alkoxygruppen mit bis zu 20 C-Atomen.

Als hydrophile Gruppe eignen sich z.B. Alkylgruppen mit 1-17 C-Atomen, die zumindest eine Hydroxylgruppe tragen und/oder funktionelle Gruppen, die beim pH der Meßlösung in dissoziiertem Zustand vorliegen, wie z.B. Carbonsäuren, Sulfonsäuren und Phorphorsäuren.

Reaktive Gruppen zur Kopplung an aminofunktionalisierte Polymere, z.B. Aminocellulose und aminofunktionelle Polyacrylamide, sind z.B. aus der US-A - 4,774,339, Tabelle 4, bekannt.

Diese oben genannten, bevorzugt eingesetzten luminophoren Reste können mit Licht einer Wellenlänge von > 450 nm angeregt werden.

Die erfindungsgemäßen Verbindungen zur Bestimmung der Alkaliionen können in gelöster Form der Probelösung zugegeben werden. Sie können aber auch Bestandteil eines Sensors sein, in welchem sie in einer Schicht aus z.B. einem Hydrogel eingebettet vorliegen, wie unten an Hand der Figur G beschrieben wird.

Die Erfindung betrifft ferner einen Diaza-Kryptand der allgemeinen Formel IV 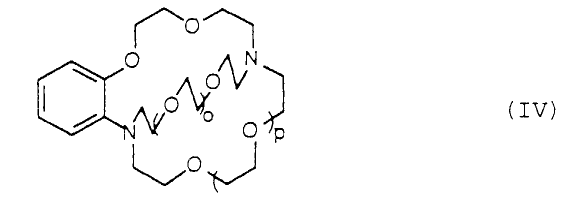 worin o und p unabhängig voneinander die Zahlen 0, 1 oder 2 bedeuten. Dieser Kryptand bildet den ionophoren Rest der erfindungsgemäßen Verbindungen der allgemeinen Formel I.

Nachfolgend wird die Erfindung beispielhaft noch näher beschrieben, wobei Synthese und Eigenschaften einiger bevorzugt verwendeter Diaza-Kryptanden erläutert werden. Andere erfindungsgemäße Verbindungen können vom Fachmann auf analoge Weise hergestellt werden.

### 1. Synthese der erfindungsgemäßen Diaza-Kryptanden

### 1.1. Synthese des ionophoren Restes der erfindungsgemäßen Diaza-Kryptanden (Figur A)

### Allgemeines Verfahren (Figur A)

Der Syntheseweg für den ionophoren Teil der erfindungsgemäßen Diaza-Kryptanden ist in der Figur A allgemein dargestellt.

2-Nitrophenol A1 wurde mit 2-Chlorethanol in n-Butanol und Natriumhydroxid zu 2'-(2'-Hydroxyethoxy)nitrobenzol A2 umgesetzt. Durch Reduktion dieser Verbindung mit Sn/SnCl₂/HCl wurde das 2-(2-Hydroxyethoxy)anilin A3 erhalten, das weiter mit 2-Chlorethanol bzw. mit 2-(2-Chlorethoxy)ethanol in Dimethylformamid in Gegenwart von Triethylamin die N-alkylierten Anilinderivate A4 mit p=0 bzw. p=1 ergaben. Durch Erwärmen von 4-Toluolsulfonamid A5 mit 2-Chlorethanol in Dimethylformamid und Kaliumkarbonat wurde das N,N-Bis(2-hydroxyethyl)-4-toluolsulfonamid A6 erhalten, das weiter mit 4-Toluolsulfonsäurechlorid das entsprechende Ditosylat A7 ergab.

Das Ditosylat A7 wurde dann mit den N-alkylierten Anilinderivaten A4 mit p=0 bzw. p=1 in t-Butanol/Tetrahydrofuran und Kalium-t-butoxid als Base zu den entsprechenden Diaza-Kronenether-Toluolsulfonamiden A8 mit p=0 bzw. p=1 umgesetzt. Die reinen Produkte wurden durch Säulenchromatographie auf Kieselgel erhalten. Die Abspaltung der Tosyl-Gruppe erfolgte mit Lithiumaluminiumhydrid in Tetrahydrofuran unter Rückfluß, wobei die Diaza-Kronenether A9 mit p=0 bzw. p=1 erhalten wurden. 3-Oxapentan-dicarbonsäuredichlorid A10 mit o=0 und 3,6-Dioxaoctandicarbonsäure-dichlorid A10 mit o=1 wurden aus den entsprechenden Dicarbonsäuren mit Oxalsäure-dichlorid in Benzol erhalten. Die Herstellung der Kryptand-bis-amide All mit o=0, p=0 bzw. o=0 und p=1 erfolgte unter hoher Verdünnung aus den Diaza-Kronenethern A9 mit p=0 bzw. p=1 durch Umsetzung mit 3-Oxapentandicarbonsäure-dichlorid A10 mit o=0 in Tetrahydrofuran. Die Kryptand-bis-amide All mit o=1, p=0 bzw. o=1, p=1 wurden analog aus den Diaza-Kronenethern A9 mit p=0 bzw. p=1 mit 3,6-Diazaoctan-dicarbonsäuredichlorid A10 mit o=1 erhalten. Die Reduktion der Amid-Gruppen mit Boran-Tetrahydrofuran-Komplex in Tetrahydrofuran ergab die Diaza-Kryptanden A12 mit o=0, p=0 bzw. o=0, p=1 und o=1, p=0 bzw. o=1, p=1.

Die Einführung der Aldehydfunktion erfolgte durch direkte Formylierung mit Phosphoroxytrichlorid in Dimethylformamid, wobei die entsprechenden Diaza-Kryptand-Aldehyde A13 mit o=0, p=0 bzw. o=0, p=1 und o=1, p=0 bzw. o=1, p=1 erhalten wurden.

Auf analoge Weise können Verbindungen A13 erhalten werden, bei welchen o und p voneinander unabhängig auch die ganze Zahl 2 bedeuten.

### Beschreibung einzelner Reaktionschritte der Figur A

### 2-(2-Hydroxyethoxy)nitrobenzol A2:

10 g (71,88 mmol) 2-Nitrophenol Al und 3,59 g (89,86 mmol) Natriumhydroxid wurden in 55 ml n-Butanol und 5 ml Wasser bei 70°C gelöst, 6,26 ml (7,52 g, 93,44 mmol) 2-Chlorethanol wurden langsam zugetropft und anschließend bei 100°C drei Tage lang kräftig gerührt. Nach Abkühlen wurde das Reaktionsgemisch filtriert, der Niederschlag mit Chloroform gewaschen und das Filtrat eingeengt. Der Rückstand wurde in Chloroform aufgenommen und drei Mal mit wässeriger 10%-iger Natriumhydroxyd-Lösung gewaschen. Dann wurde die organische Phase über Natriumsulfat getrocknet und im Vakuum konzentriert. Es wurden 10,8 g hellgelbe Kristalle erhalten; Ausbeute 82%.

¹H-NMR (CDCl₃), δ (ppm): 3,97 (m, 3H), 4,22 (t, 2H), 6,98-7,13 (m, 2H), 7,52 (m, 1H) , 7,83 (m, 1H).

### 2-(2-Hydroxyethoxy)anilin A3:

8,9 g (48,59 mmol) 2-(2-Hydroxyethoxy)nitrobenzol A2, 16,44 g (72,88 mmol) SnCl₂.2H₂O und 17,3 g (145,77 mmol) Zinn wurden in 30,84 ml wässeriger HCl (30%) und 25 ml Wasser bei 90°C 8 Stunden gerührt. Die Lösung wurde nach Abkühlen mit wässeriger 5n Natriumhydroxyd-Lösung behandelt und bei 90°C 3 Stunden gerührt. Die wässerige Lösung wurde anschließend dekantiert, wobei nach Abkühlen das Rohprodukt auskristallisierte und abgesaugt wurde. Dieses wurde dann in Methanol aufgenommen, erwärmt und die Suspension filtriert. Das Filtrat wurde dann im Vakuum eingeengt. Es wurden 6 g hellbraune Kristalle erhalten; Ausbeute 80%.

¹H-NMR (CDCl₃), δ (ppm): 3,62 (m, 3H), 3,97 (t, 2H), 6,52-6,75 (m, 4H).

### N-[2-(2-Hydroxyethoxy)ethyl]-2-(2-hydroxyethoxy)anilin A4 (p=1) :

6 g (39,17 mmol) 2-(2-Hydroxyethoxy)anilin A3, 5 ml (5,85 g, 47 mmol) 2-(2-Chlorethoxy)ethanol und 8,19 ml (5,95 g, 58,75 mmol) Triethylamin wurden in 20 ml Dimethylformamid gelöst und 4 Tage bei 90°C gerührt. Nach Abkühlen wurde die Lösung filtriert, der Niederschlag mit Dichlormethan gewaschen und das Filtrat im Vakuum konzentriert. Der Rückstand wurde in Chloroform aufgenommen und 2 mal mit wenig Wasser gewaschen. Die Chloroform-Lösung wurde dann über Natriumsulfat getrocknet und eingeengt. Das zähflüssige Rohprodukt wurde durch Säulenchromatographie auf Kieselgel 60 mit Toluol/Aceton 1:2 als Laufmittel gereinigt. Es wurden 5 g braunes, zähflüssiges Öl erhalten; Ausbeute 53%.

¹H-NMR (CDCl₃), δ (ppm): 3,26 (m, 2H), 3,52 (m, 2H), 3,67 (m, 4H), 3,83 (m, 2H), 4,00 (m, 2H), 4,56 (br. s, 2H), 6,55-6,95 (m, 4H).

### N,N-Bis (2-hydroxyethyl)-4-toluolsulfonamid A6:

6,84 g (40 mmol) 4-Toluolsulfonamid A5, 7 ml (8,37 g, 104 mmol) 2-Chlorethanol und 27,64 g (200 mmol) Kaliumkarbonat wurden in 100 ml Dimethylformamid suspendiert und 3 Tage bei 110°C gerührt. Nach Abkühlen wurde das Reaktionsgemisch filtriert und der Niederschlag mit Chloroform gewaschen. Das Filtrat wurde eingeengt, der ölige Rückstand in Chloroform aufgenommen und schließlich mit einer 10%-igen Natriumhydroxyd-Lösung gewaschen. Nach Konzentrieren der organischen Lösung wurden 8,4 g reines Produkt als hellgelbe Kristalle erhalten; Ausbeute: 81%.

¹H-NMR (CDCl₃), δ (ppm): 2,39 (s, 3H), 3,21 (t, 4H), 3,82 (t, 4H), 4,50 (br s, 2H), 7,29 (d, 2H), 7,65 (d, 2H).

### N,N-Bis(2-hydroxyethyl)-4-toluolsulfonamid-bis-toluolsulfonat A7:

4,54 g (17,5 mmol) N,N-Bis(2-hydroxyethyl)-4-toluolsulfonamid A6 wurden in 20 ml Aceton gelöst und auf -5°C abgekühlt. Danach wurden 8 g (42 mmol) 4-Toluolsulfonsäurechlorid dazugegeben und 10 min gerührt. Eine wässerige 25%-ige Natriumhydroxyd-Lösung wurde bei -2°C langsam zugetropft, dann wurde weiter 8 h bei 0°C gerührt und das Reaktionsgemisch über Nacht in den Kühlschrank gestellt. Das Gemisch wurde über Eiswasser geschüttet, wobei das Produkt als zähflüssiges Öl ausfiel. Die wässerige Phase wurde vorsichtig dekantiert, das Produkt in Chloroform aufgenommen und mit Wasser gewaschen. Nach Einengen des Lösungsmittels wurden 9,2 g reines Produkt als hellgelbes, zähflüssiges Öl, das in der Kälte langsam kristallisierte, erhalten; Ausbeute: 92%.

¹H-NMR (CDCl₃), δ (ppm): 2,41 (s, 3H), 2,46 (s, 3H), 3,36 (t, 4H), 4,10 (t, 4H), 4,50 (br s, 2H), 7,29 (d, 2H), 7,34 (d, 4H), 7,62 (d, 2H), 7,76 (d, 4H).

### Diaza-Kronenether-Toluolsulfonamid A8 (p=1) :

5 g (20,72 mmol) Anilinderivat A4 (p=1) und 6,05 g (53,88 mmol) Kalium-t-butoxid wurden in 280 ml t-Butanol unter Stickstoff gelöst und 2h bei 60°C gerührt. Dann wurden 11,76 g (20,72 mmol) Toluolsulfonat A7 in 140 ml trockenem Tetrahydrofuran während 2h bei 40°C zugetropft. Das Reaktionsgemisch wurde anschließend 48h bei 60°C gerührt. Nach Abkühlen wurde die Lösung filtriert, der Niederschlag mit Dichlormethan gewaschen und das Filtrat im Vakuum eingeengt. Der Rückstand wurde in Chloroform gelöst und 2 mal mit Wasser gewaschen. Die organische Lösung wurde schließlich konzentriert, wobei sich ein dunkler, öliger Rückstand bildete. Aus diesem wurde das Produkt durch Säulenchromatographie auf Kieselgel und Toluol/Aceton 10:9 als Laufmittel erhalten: 1 g gelbes, zähflüssiges Öl; Ausbeute: 10%.

¹H-NMR (CDCl₃), δ (ppm): 2,40 (s, 3H), 3,20-4,50 (m, 20H), 6,55-6,96 (m, 4H), 7,27 (d, 2H), 7,67 (d, 2H).

### Diaza-Kronenether A9 (p=1) :

0,41 g (10,8 mmol) Lithiumaluminiumhydrid wurden in 10 ml trockenem Tetrahydrofuran unter Stickstoff suspendiert, und 0,5 g (1,08 mmol) Diaza-Kronenether-Toluolsulfonamid A8 (p=1) in 10 ml Tetrahydrofuran wurden langsam zugetropft. Das Reaktionsgemisch wurde 3 Tage unter Rückfluß gerührt. Nach Abkühlen wurde überschüssiges Lithiumaluminiumhydrid mit Tetrahydrofuran/Wasser 2:1 (v/v) zersetzt, filtriert und der Niederschlag mit Dichlormethan gewaschen. Der Rückstand wurde in Dichlormethan aufgenommen, filtriert und das Lösungsmittel im Vakuum entfernt. Es wurden 0,25 g hellbraunes Öl erhalten; Ausbeute: 75%.

### Kryptand-bis-amid A11 (0=1, p=1) :

Diese Reaktion wurde nach der Methode von R. Crossley, Z. Goolamali, P.G. Sammes, J. Chem. Soc. Perkin Trans. 2, 1994, 1615-1622 durchgeführt. Das Dicarbonsäure-dichlorid A10 (o=0) wurde nach der Methode von B. Dietrich, J.M. Lehn, J.P. Sauvage, J. Blanzat, Tetrahedron 1973, 29, 1629-1645 erhalten.

0,13 g (1,67 mmol) Pyridin wurden in 240 ml trockenem Tetrahydrofuran gelöst und auf 0°C abgekühlt. 0,25 g (0,805 mmol) Diaza-Kronenether A9 (p=1) in 40 ml trockenem Tetrahydrofuran und 0,17 g (0,605 mmol) 3,6-Dioxaoctandicarbonsäure-dichlorid A10 (o=1) in 40 ml trockenem Tetrahydrofuran wurden dann gleichzeitig während 4 Stunden zugetropft. Das Reaktionsgemisch wurde dann weitere 40 Stunden bei 0°C gerührt. Das Gemisch wurde filtriert und das Tetrahydrofuran entfernt. Der ölige, braune Rückstand wurde in Chloroform gelöst, mit verdünnter HCl und dann mit Wasser gewaschen. Die Chloroform-Lösung wurde über Natriumsulfat getrocknet und konzentriert. Es wurde ein öliger, hellbrauner Rückstand erhalten; Ausbeute: 0,12 g (33%).

### Diaza-Kryptand A12 (o=1, p=1) :

In einem Dreihalskolben, versehen mit Stickstoffzufuhr, Septum und Rückflußkühler mit Kalziumchlorid-Röhrchen, wurden 0,12 g (0,27 mmol) Kryptand-bis-amid A11 (o=1, p=1) vorgelegt. 2,5 ml trockenes Tetrahydrofuran wurden durch das Septum mit Hilfe einer Spritze dazugegeben, und die Suspension wurde in einem Eisbad gekühlt. 2,2 ml (2,2 mmol) einer 1 m Boran-THF-Komplex-Lösung wurden dann vorsichtig ebenfalls durch das Septum zugegeben. Nach 10 min wurde das Eisbad entfernt, das Reaktionsgemisch bei 30 min bei Raumtemperatur und anschließend 2 Stunden unter Rückfluß gerührt. Nach Abkühlen wurde das Gemisch vorsichtig mit 1 ml Wasser und 10 ml wässeriger 6 n HCl versetzt und 1 Stunde bei Raumtemperatur gerührt. Das Lösungsmittel wurde im Vakuum entfernt und die erhaltene weiße Suspension mit wässeriger Lithiumhydroxid-Lösung auf pH 10 gebracht. Die wässerige Phase wurde mit Chloroform extrahiert. Nach Konzentrieren der Lösung im Vakuum wurde ein hellgelbes Öl erhalten; Ausbeute: 0,1 g (88%).

### Diaza-Kryptand-Aldehyd A13 (o=1, p=1) :

0,5 g (1,18 mmol) Diaza-Kryptand A12 (0=1, p=1) wurden in 1,5 ml Dimethylformamid gelöst und auf -10°C gekühlt. 0,22 ml (0,36 g, 2,35 mmol) Phosphoroxytrichlorid wurden vorsichtig zugetropft, so daß die Temperatur 0°C nicht überschritt. Das Reaktionsgemisch wurde 15 min bei -5°C, dann über Nacht bei Raumtemperatur und schließlich 1 Stunde bei 60°C gerührt. Nach Abkühlen wurde das Gemisch mit Wasser versetzt, mit einer wässerigen, konzentrierten Lithiumhydroxid-Lösung auf pH 9 gebracht und 30 min gerührt. Die Lösung wurde 3 mal mit je 30 ml Chloroform extrahiert und die organische Phase im Vakuum konzentriert. Es wurden 0,32 g gelbes Öl erhalten; Ausbeute: 60%.

### 1.2 Herstellung der erfindungsgemäßen Diaza-Kryptanden (Figuren B und C)

Die Herstellung der erfindungsgemäßen Diaza-Kryptanden ist in den Figuren B und C für zwei luminophore Reste (Amino-Naphthalimid bzw. Xanthenon) beispielhaft dargestellt, wobei "Y" jeweils für einen ionophoren Rest steht.

### Allgemeines Verfahren der Figur B (der luminophore Rest ist ein Amino-Naphthalimid-Rest)

Erfindungsgemäße Verbindungen mit einer einzigen CH₂-Gruppe als Spacer zwischen dem luminophoren und dem ionophoren Rest wurden hergestellt, indem zunächst die Verbindung C1, welche beispielsweise die Verbindung A13 (Fig. A) sein kann, in der "Y" für A12 steht, in das Oxim C2 übergeführt und mittels Zn in Essigsäure zum Amin C3 reduziert wurde. Die Kopplung an den luminophoren Rest zur Herstellung der erfindungsgemäßen Verbindung C9 ist in der Figur B an Hand des Amino-Naphthalimids C8, welches durch Umsetzung von C6 mit C7 in Dimethylformamid in Gegenwart von K₂CO₃ hergestellt wurde, schematisch gezeigt.

Erfindungsgemäße Verbindungen mit zwei CH₂-Gruppen als Spacer zwischen dem luminophoren und dem ionophoren Rest wurden hergestellt, indem zunächst die Verbindung C1, mit einem großen Überschuß an Nitromethan in der Gegenwart von Ammoniumacetat zur Verbindung C4 umgesetzt wurde, die anschließend mittels LiAlH₄ in THF zum Amin C5 reduziert wurde. Die Kopplung an das Amino-Naphthalimid C8 zur Herstellung der erfindungsgemäßen Verbindung C10 ist in der Figur B ebenfalls schematisch gezeigt.

### Beschreibung einzelner Reaktionschritte der Figur B

### 4-Chlor-N-(4-carboxyphenylmethyl)-1,8-naphthalimid C8:

46,4 g (200 mmol) 4-Chlor-1,8-naphthalinsäureanhydrid C7, 30,2 g (200 mmol) 4-Aminomethylbenzoesäure C6 und 13,8 g (100 mmol) K₂CO₃ wurden in 2 l Dimethylformamid suspensdiert, bei Raumtemperatur 16 Stunden und bei 60°C 6 Stunden gerührt. Das Gemisch wurde anschließend in 4 l Wasser gegossen und mit 6 n HCl auf pH 4 gebracht. Das erhaltene Präzipitat wurde abfiltriert und 18 min bei 60°C getrocknet, wobei 36 g schmutzig weißes Pulver erhalten wurden (Ausbeute: 51%).

### Diaza-Kryptand-Aldoxim C2 (o=1, p=1) :

1 g (2,21 mmol) Diaza-Kryptand-Aldehyd A13 mit o=1 und p=1 wurde in 10 ml Ethanol und 7 ml Wasser suspendiert. Dann wurden 0,31 g (4,42 mmol) Hydroxylamin-hydrochlorid und 0,26 g (2,43 mmol) Na₂CO₃ zugegeben. Das Reaktionsgemisch wurde 5 Stunden auf 60°C erwärmt. Nach Abkühlen der Lösung wurde mit Eiswasser versetzt. Das Oxim setzte sich als Öl ab, wurde in Chloroform aufgenommen und mit Wasser gewaschen. Nach Trocknen und Einengen der organischen Lösung wurden 0,83 g Oxim als hellgelbes Öl erhalten (Ausbeute: 80%) .

### Diaza-Kryptand-Amin C3 (o=1, p=1) :

0,83 g (1,77 mmol) Diaza-Kryptand-Aldoxim C2 mit o=1 und p=1 wurden in 12 ml Essigsäure gelöst, und 1,62 g (24,75 mmol) Zink wurden zugegeben. Das Reaktionsgemisch wurde über Nacht bei Raumtemperatur und anschließend 3 Stunden bei 65°C gerührt. Nach Abkühlen wurde das Gemisch mit Dichlormethan versetzt und filtriert, um überschüssiges Zink und Zinksalze zu entfernen. Der Niederschlag wurde mit Dichlormethan gewaschen. Das Filtrat wurde im Vakuum konzentriert, der gelbe Rückstand wurde anschließend mit Wasser versetzt und mit wässeriger Lithiumhydroxid-Lösung auf pH 9 gebracht. Das Produkt wurde dann mit Dichlormethan aus der wässerigen Lösung extrahiert, und das Lösungsmittel wurde im Vakuum entfernt. Es wurden 0,56 g dunkelgelbes Öl erhalten (Ausbeute: 70%).

### 4-(2-Nitroethenyl)-Diaza-Kryptand C4 (o=1, p=1) :

1 g (2,21 mmol) Diaza-Kryptand-Aldehyd A13 mit o=1 und p=1 wurde in 4,5 ml Essigsäure gelöst und bei Raumtemperatur 10 min gerührt. Dann wurden 2,63 ml (2,97 g, 48, 62 mmol) Nitromethan zugegeben und 5 h bei 60°C gerührt. Nach Abkühlen wurde das Gemisch auf Eiswasser gegossen, wobei ein dunkelroter Niederschlag entstand, der abgesaugt und mit Wasser gewaschen wurde. Nach Trocknen im Exsikkator wurden 0,6 g Produkt (Ausbeute: 55%) erhalten.

### 4-(2-Aminoethyl)-Diaza-Kryptand C5 (o=1, p=1) :

In einem Dreihalskolben, versehen mit Stickstoffzufuhr und Rückflußkühler mit Kalziumchlorid-Röhrchen, wurden 0,46 g (12,1 mmol) LiAlH₄ in 25 ml trockenem Tetrahydrofuran suspendiert. Dann wurden 0,6 g (1,21 mmol) 4-(2-Nitroethenyl)-Diaza-Kryptand C4 mit o=1 und p=1 in 6 ml trockenem Tetrahydrofuran langsam zugetropft. Anschließend wurde das Gemisch 4 Stunden unter Rückfluß gerührt. Das Reaktionsgemisch wurde in einem Eisbad gekühlt, und überschüssiges LiAlH₄ wurde mit einem Gemisch aus Tetrahydrofuran und wässeriger NaOH vorsichtig zersetzt. Das Gemisch wurde filtriert, der Niederschlag mit Chloroform gewaschen und das Filtrat eingeengt. Der Rückstand wurde in Chloroform aufgenommen, mit Wasser gewaschen und die Lösung konzentriert, wobei 0,45 g dunkelgelbes Öl erhalten wurden (Ausbeute: 80%) .

### Diaza-Kryptand-Fluoroionophor C9 (o=1, p=1) :

0,56 g (1,23 mmol) Diaza-Kryptand-Amin C3 mit o=1 und p=1, 0,42 g (1,23 mmol) 4-Chlor-naphthalimidderivat C8 und 0,4 g (3,09 mmol) Diisopropylethylamin wurden in 2,5 ml N-Methylpyrrolidinon suspendiert. Das Reaktionsgemisch wurde unter Stickstoff 24 Stunden bei 60°C gerührt. Nach Abkühlen wurde die gelbe Lösung mit Wasser versetzt, wobei das Produkt zusammen mit nicht umgesetztem 4-Chlor-naphthalimidderivat ausfiel. Der Niederschlag wurde filtriert, mit Wasser gewaschen und im Exsikkator über Nacht getrocknet. Das Gemisch wurde dann in warmem Chloroform/Methanol (3:1) aufgenommen, filtriert, die Lösung konzentriert und der Rückstand mittels Säulenchromatographie auf Kieselgel und Chloroform/Methanol (3:1) und 1% Essigsäure als Laufmittel gereinigt. Es wurden 0,065 g Produkt (Ausbeute: 7%) erhalten.

### Diaza-Kryptand-Fluoroionophor C10 (o=1, p=1) :

0,45 g (0,96 mmol) 4-(2-Aminoethyl)-Diaza-Kryptand C5 mit o=1 und p=1, 0,33 g (0,96 mmol) 4-Chlor-naphthalimidderivat C8 und 0,31 g (2,4 mmol) Diisopropylethylamin wurden in 2,5 ml N-Methylpyrrolidinon suspendiert. Das Reaktionsgemisch wurde unter Stickstoff 24 Stunden bei 60°C gerührt. Nach Abkühlen wurde die gelbe Lösung mit Wasser versetzt, wobei das Produkt zusammen mit nicht umgesetztem 4-Chlor-naphthalimidderivat ausfiel. Der Niederschlag wurde filtriert, mit Wasser gewaschen und im Exsikkator über Nacht getrocknet. Das Gemisch wurde dann in warmem Chloroform/Methanol (3:1) aufgenommen, filtriert, die Lösung konzentriert und der Rückstand mittels Säulenchromatographie auf Kieselgel und Chloroform/Methanol (3:1) und 1% Essigsäure als Laufmittel gereinigt. Es wurden 0,074 g Produkt (Ausbeute: 10%) erhalten.

### Verfahren der Figur C (der luminophore Rest ist ein Xanthenon-Rest)

### Diaza-Kryptand-2,3,7-trihydroxyfluoron D3 (o=1, p=1) :

Einer Suspension von 1,67 g (6,63 mmol) 1,2,4-Triacetoxybenzol D2 in 16 ml 50%-igem Ethanol wurden 1,4 ml konzentrierte Schwefelsäure zugetropft, wonach 10 min gerührt und dann 1,5 g (3,31 mmol) Diaza-Kryptand-Aldehyd D1 (=A13) zugegeben wurden. Die Suspension wurde dann 24 Stunden bei 80°C gerührt. Das Gemisch wurde dann mit Wasser versetzt, mit 25%-igem Tetramethylammoniumhydroxid auf pH 5 gebracht und über Nacht stehengelassen. Das Reaktionsgemisch wurde dekantiert, der Rückstand mit Wasser gewaschen und anschließend mit Methanol versetzt und gerührt. Nach Entfernen des Lösungsmittels im Vakuum wurden 0,67 g rotes Produkt erhalten (Ausbeute: 30%).

### Diaza-Kryptand-2,3,7-tri-t-butoxycarbonylmethoxyfluoron D4 (o=1, p=1) :

0,67 g (1 mmol) Diaza-Kryptand-2,3,7-trihydroxyfluoron D3 mit o=1 und p=1, 0,45 g (3 mmol) Natriumjodid, 0,48 g (4,5 mmol) Natriumkarbonat und 0,88 g (4,5 mmol) t-Butylbromacetat wurden in 5 ml Dimethylformamid suspendiert und 1 Stunde bei 110°C gerührt. Nach Abkühlen wurde das Gemisch mit Wasser verdünnt und mit Chloroform extrahiert. Die organische Lösung wurde mit Wasser gewaschen, über Natriumsulfat getrocknet und konzentriert. Das Rohprodukt wurde dann mittels Säulenchromatographie auf Kieselgel mit Chloroform/Methanol (9:1) gereinigt, wobei 0,55 g rotes, zähflüssiges Produkt erhalten wurden (Ausbeute: 55%).

### Diaza-Kryptand-2,3,7-tri-carboxymethoxyfluoron D5 (o=1, p=1) :

0,55 g (0,54 mmol) Diaza-Kryptand-2,3,7-tri-t-butoxycarbonylmethoxyfluoron D4 mit o=1 und p=1 wurden in 2 ml Dichlormethan gelöst. 0,25 ml Trifluoressigsäure wurden zugegeben und 4 Stunden wurde bei 40°C gerührt. Das Reaktionsgemisch wurde dann im Vakuum konzentriert und der Rückstand in Methanol gelöst. Das Lösungsmittel wurde teilweise abgedampft und der Vorgang 2 mal wiederholt, um die Trifluoressigsäure möglichst vollständig zu entfernen. Es wurden 0,36 g rotes, zähflüssiges Produkt erhalten (Ausbeute 90%).

### 2. Lumineszenzeigenschaften der erfindungsgemäßen Diaza-Kryptanden

Die Figuren D, E und F zeigen die Lumineszenzeigenschaften von erfindungsgemäßen Verbindungen, immobilisiert auf Cellulose, in Abhängigkeit von der jeweiligen Konzentration an Alkaliionen. Die Ordinaten der dargestellten Diagramme geben jeweils die relativen Lumineszenzintensitäten an.

### Figur D

Die Fig. D wurde mit dem Diaza-Kryptanden C9 erhalten.

Die Immobilisierung auf Cellulosefasern (aminomodifiziert) wurde wie folgt vorgenommen:

0,03 mmol des Diaza-Kryptanden C9, 0,06 g (0,3 mmol)
N,N-Dicyclohexyl-1,3-carbodiimid, 0,4 g (0,3 mmol) N-Hydroxysuccinimid und 5 g aktivierte Cellulose (hergestellt gemäß SU-A - 1,028,677, CA 99:177723h) wurden in 2 ml Dimethylformamid 20 Stunden suspendiert. Anschließend wurde die Cellulose abfiltriert, 5 mal mit 5 ml Dimethylformamid, 5 ml Wasser, 2 mal mit 5 ml 0,2 n HCl, 5 ml Wasser, 2 mal mit 5 ml 0,2 n NaOH, 10 mal mit 5 ml Wasser, 2 mal mit 5 ml Aceton und 2 mal mit 5 ml Ether gewaschen, und 16 Stunden bei Raumtemperatur getrocknet. Anschließend wurde die Cellulose gesiebt (25 µm).

Sensorscheiben wurden wie folgt hergestellt:

0,25 g gesiebte (25 µm) Aminocellulosefasern mit immobilisiertem Kronenether C9 wurden in 4,75 g 10% Hydrogel D4 (Tyndale Plains-Hunter LTD. Ringoes, NJ 08551) in 90% Ethanol-Wasser 16 Stunden suspendiert. Die erhaltene homogene Dispersion wurde auf eine Polyesterfolie (Melinex-Folie, ICI America) bis zu einer Trockendichte von 10 µm aufgetragen. Diese Folie wurde mit 3% Aktivkohle in 10% D4-Hydrogel bis zu einer Trockendichte von 5 µm überschichtet, worauf eine kleine Scheibe mit einem Durchmesser von 2,5 cm herausgeschnitten wurde. Diese Scheibe wurde zur Aktivierung mindestens 16 Stunden im Puffer gelassen.

Eine Methode zum Schneiden und Messen von Sensorscheiben wurde von M.J.P. Leiner und P. Hartmann in Sensors and Actuators B, 11 (1993), 281-289 ("Theory and practice in optical pH sensing") beschrieben.

Die erhaltene Sensorscheibe wurde in der in der Figur G schematisch dargestellten Meßanordnung verwendet.

In der Figur G steht das Bezugszeichen S für einen Abschnitt der Sensorscheibe. Die an den Cellulosefasern immobilisierte Verbindung ist mit I bezeichnet und liegt im Hydrogel (Schicht M) vor. Diese Schicht M ist ionenpermeabel und wird von einem für die Anregungs- und Meßstrahlung durchlässigen Träger T, der eine transparente Folie ist, getragen.

Die Verbindung I kann erfindungsgemäß an die ionenpermeable Matrix direkt kovalent gebunden sein oder in der Matrix physikalisch gelöst vorliegen.

Zur Messung wurde die Sensorscheibe in eine lichtundurchlässige, thermostatisierte Durchflußzelle eingebracht und mit Proben P, die unterschiedliche Natriumionenkonzentrationen aufwiesen, in Kontakt gebracht.

Die optische Meßeinrichtung bestand aus einer blauen LED als Lichtquelle A, einer Photodiode M als Detektor, optischen Filtern A und F zur Auswahl der Wellenlängen, einer faseroptischen Anordnung zur Leitung des Anregungslichtes in die Polymerschicht M und zur Leitung des Emissionslichtes zum Photodetektor M, sowie einer Einrichtung zur elektronischen Signalverarbeitung (nicht dargestellt). Anregungsseitig wurde ein Interferenzfilter (Peak-Transmission bei 480 nm) und emissionsseitig ein 520 nm cut-off Kantenfilter verwendet.

Die Figur D zeigt die relative Lumineszenzintensität (Ordinate) in Abhängigkeit von verschiedenen Kaliumionen-Konzentrationen (0,2, 0,6, 1, 3, 5, 7, 9, 20, 50 und 100 mmol/l; Abszisse; logarithmische Skala), jeweils in Gegenwart von 145 mmol/l Natriumionen. Das Meßmedium war 30 mmol/1 Tris/HCl-Puffer, CO₂-frei; pH: 7,4; 37°C.

### Figur E

Die Figur E wurde analog Figur D erhalten, wobei jedoch anstelle des Diaza-Kryptanden C9 der Diaza-Kryptand C10 verwendet wurde und die Lumineszenzintensität in Abhängigkeit von verschiedenen Kaliumionen-Konzentrationen (0,2, 0,6, 1, 3, 5, 7, 9, 20, 50 und 100 mmol/l; Abszisse; logarithmische Skala), jeweils in Gegenwart von 145 mmol/l Natriumionen, gemessen wurde.

### Figur F

Die Figur F wurde analog Figur D erhalten, wobei jedoch anstelle des Diaza-Kryptanden C9 der Diaza-Kryptand D5 verwendet wurde und die Lumineszenzintensität in Abhängigkeit von verschiedenen Kaliumionen-Konzentrationen (0,1, 0,5, 1, 3, 5, 10 und 50 mmol/l; Abszisse; logarithmische Skala), jeweils in Gegenwart von 145 mmol/l Natriumionen, gemessen wurde.

## Patentansprüche

1. Verfahren zur Bestimmung eines Alkaliions in einer Probe, wobei das Alkaliion mit einer Verbindung, die einen luminophoren Rest und einen ionophoren Rest aufweist, in Kontakt gebracht wird, welcher ionophore Rest mit dem in der Probe enthaltenen Alkaliion reagiert, wobei der luminophore Rest seine Lumineszenzeigenschaften ändert, wonach die Lumineszenz gemessen und mit dem Meßergebnis das Alkaliion bestimmt wird,
dadurch gekennzeichnet, daß
als Verbindung ein Diaza-Kryptand der allgemeinen Formel I 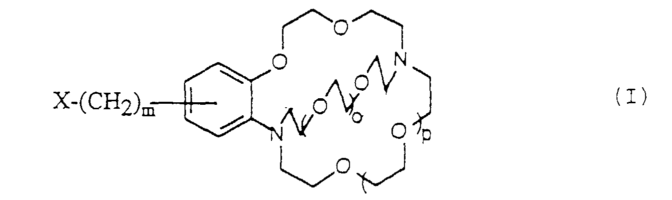 worin X der luminophore Rest ist, m die Zahl 0, 1 oder 2 ist, und o und p unabhängig voneinander die Zahlen 0, 1 oder 2 bedeuten, eingesetzt wird.

2. Verfahren nach Anspruch 1, dadurch gekennzeichnet, daß zur Bestimmung von Lithiumionen ein Diaza-Kryptand der allgemeinen Formel I eingesetzt wird, in welcher o und p die Zahlen 0 bzw. 0 bedeuten.

3. Verfahren nach Anspruch 1, dadurch gekennzeichnet, daß zur Bestimmung von Natriumionen ein Diaza-Kryptand der allgemeinen Formel I eingesetzt wird, in welcher o und p die Zahlen 0 bzw. 1 bedeuten, oder die Zahlen 1 bzw. 0 bedeuten.

4. Verfahren nach Anspruch 1, dadurch gekennzeichnet, daß zur Bestimmung von Kaliumionen ein Diaza-Kryptand der allgemeinen Formel I eingesetzt wird, in welcher o und p die Zahlen 1 bzw. 1 bedeuten.

5. Verfahren nach einem der Ansprüche 1 bis 4, dadurch gekennzeichnet, daß der luminophore Rest X in der allgemeinen Formel I
- ein Amino-Naphthalimid-Rest der allgemeinen Formel II 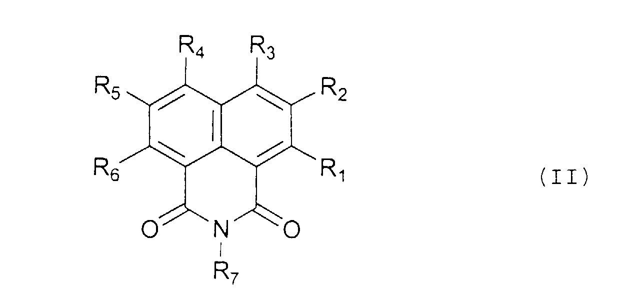 ist, in welcher eine von R₁, R₂, R₃, R₄, R₅ und R₆ eine Gruppe -NH- ist, über welche X an die Gruppe -(CH₂)ₘ- der im Anspruch 1 genannten Verbindung mit der allgemeinen Formel I gebunden ist, und die anderen und R₇ jeweils voneinander unabhängig Wasserstoff, eine lipophile oder hydrophile Gruppe oder eine reaktive Gruppe zur Kopplung an ein Polymer sind,
oder
- ein Xanthenon-Rest der allgemeinen Formel III 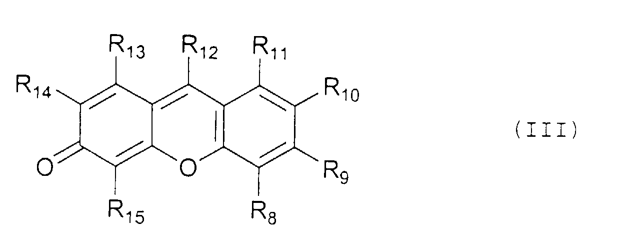 ist, in welcher eine von R₈, R₉, R₁₀, R₁₁, R₁₂, R₁₃, R₁₄ und R₁₅ für eine chemische Bindung steht, über welche X direkt (m=0) an den ionophoren Rest der im Anspruch 1 genannten Verbindung mit der allgemeinen Formel I gebunden ist, und die übrigen für -OH, -OR₁₆, worin R₁₆ eine hydrophile oder eine lipophile Gruppe ist, -O-R₁₇-G, worin R₁₇ eine hydrophile oder eine lipophile Gruppe und G eine reaktive Gruppe zur Kopplung an ein Polymer ist, oder -(CH₂)ₙ-COOH, worin n eine ganze Zahl zwischen 0 und 17 ist, stehen.

6. Diaza-Kryptand der allgemeinen Formel I 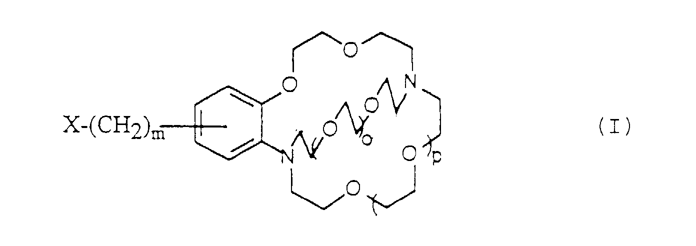 worin X der luminophore Rest ist und insbesondere die in Anspruch 5 angegebene Bedeutung besitzt, m die ganze Zahl 0, 1 oder 2 ist, und o und p unabhängig voneinander die Zahlen 0, 1 oder 2 bedeuten.

7. Diaza-Kryptand der allgemeinen Formel IV 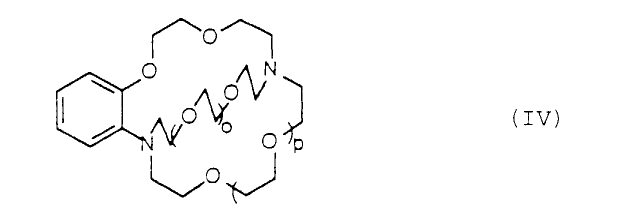 worin o und p unabhängig voneinander die Zahlen 0, 1 oder 2 bedeuten.
